# FASCICULE DE BREVET EUROPEEN

(11) **EP 2 394 613 B1**
(45) Date de publication et mention de la délivrance du brevet: **04.11.2015**
(21) Numéro de dépôt: 11169543.3
(22) Date de dépôt: 10.06.2011
(51) Int. Cl.: A61F 5/02

(54) **Orthèse orthopédique évolutive**
Erweiterbare orthopädische Orthese
Adaptive orthopaedic orthosis

(30) Priorité: 11.06.2010 FR 1054669
(43) Date de publication de la demande: 14.12.2011
(73) Titulaire: RICHARD FRERES, 42530 Saint Genest Lerpt (FR)
(72) Inventeur: Richard, Dominique, 42110 Aurec/Loire (FR); Faure, Didier, 43160 Saint Didier en Velay (FR)
(74) Mandataire: Delorme, Nicolas

(56) Documents cités:
- DE-A1- 10 329 454
- US-A- 5 038 760
- US-A- 5 722 940
- US-A1- 2006 149 180

## Description

La présente invention concerne une orthèse orthopédique à contention évolutive.

Les troubles musculo-squelettiques sont des pathologies très répandues qui trouvent leur origine dans des activités sportives, professionnelles ou de loisirs.

Les orthèses orthopédiques dont le port est prescrit pour ce type de pathologies - qu'elles agissent au niveau du cou, du dos, du poignet, de l'épaule, du coude, du genou ou de la cheville - présentent généralement une structure figée.

En d'autres termes, quelle que soient leurs applications, les orthèses de l'état de la technique sont adaptées à un stade de l'évolution de la pathologie.

En revanche, elles s'avèrent inadaptées pour suivre l'évolution de la pathologie depuis une phase aigüe jusqu'à une phase de guérison où le port de l'orthèse peut être suspendu.

Par ailleurs, le caractère figé des orthèses de l'art antérieur ne favorise pas l'observance. En effet, une orthèse prescrite, par exemple, pour une lombalgie avec un rappel de posture prononcée risque de voir son port interrompu de manière prématurée puisque l'utilisateur pourra ressentir une inadéquation entre l'orthèse en question et l'évolution de sa pathologie.

D'autre part, certaines pathologies peuvent être traitées de manière plus efficace par des dispositifs qui sont différents lors d'un port nocturne et d'un port diurne dans le but de favoriser une guérison plus rapide.

On voit donc que les dispositifs orthopédiques de l'art antérieur sont essentiellement de structure statique ce qui présente de nombreux inconvénients, bien qu'il soit connu du document de brevet US5038760 un vêtement de soutien orthopédique comprenant des panneaux avant et arrière de matériau en tissu non élastique munis à demeure de baleines flexibles pour soutenir le dos et l'abdomen d'un utilisateur, le panneau arrière étant adapté pour recevoir de façon amovible des baleines rigides afin de fournir un soutien supplémentaire en cas de besoin.

Dans ce contexte technique, la présente invention a pour but de pallier tout ou partie des problèmes précités. La présente invention a notamment pour but de proposer une orthèse orthopédique qui puisse être adaptée aux évolutions de pathologie et/ou aux évolutions de prescription.

La présente invention concerne une orthèse orthopédique telle que revendiquée dans la revendication 1.

Il est aussi connu du document de brevet US5722940 un appui-dos pour travailleurs comprenant notamment un rembourrage lombo-sacré, qui peut être positionné à l'intérieur d'une poche située sur une partie arrière du dispositif.

On notera également qu'il est connu du document de brevet US2006/0149180 un gant orthopédique avec fixation support de poignet pour traiter la perte ou la dépréciation des extenseurs et/ou la fonction des muscles fléchisseurs dans les mains et les doigts. Le gant comprend des canaux dans lesquels des éléments élastiques extenseurs interchangeables sont agencés. Ces éléments peuvent être remplacés pour adapter la résilience du gant en fonction de l'évolution de l'état du patient.

Il est en outre connu du document de brevet DE10329454 une ceinture de soutien lombaire comprenant un module supérieur avec une extrémité en forme de fourche inférieure, qui peut être fixée le cas échéant à un module élastique inférieur enroulé autour de la taille d'un utilisateur et pourvu de plusieurs bandes de renforcement. Un soutien supplémentaire peut être fourni par un insert plat flexible intégré dans une zone prévue à cet effet.

Ainsi, l'invention permet de paramétrer une orthèse en lui adjoignant un ou plusieurs éléments de rappel de posture qui peuvent se superposer pour un effet de rappel maximal ou qui peuvent être mis en place de manière indépendante pour un effet de rappel modulé. L'orthèse selon l'invention permet donc de suivre l'évolution d'une pathologie ou d'être adaptée à des prescriptions de port différentes, par exemple, de port diurne et nocturne.

De préférence, ladite orthèse comprend au moins deux fourreaux de textile disposés à distance l'une de l'autre.

Selon une possibilité, au moins un fourreau de réception est constitué d'au moins deux bandes de textile superposées fixées sur le corps principal de manière à délimiter au moins deux gaines dans chacune desquelles une baleine élastique de rappel de posture peut être indépendamment engagée.

Selon une possibilité, le fourreau intègre au moins deux gaines qui présentent des dimensions différentes de manière à recevoir des baleines de rappel de posture de caractéristiques dimensionnelles et constructives différentes.

Selon une autre possibilité, l'orthèse comprend un plastron équipé d'au moins deux baleines de rappel conçues pour s'engager sur une gaine de deux fourreaux de manière à juxtaposer le plastron et le corps principal pour augmenter la surface d'appui de l'orthèse.

Selon la présente invention, au moins un fourreau de réception comprend une bande de textile rapportée sur le corps principal qui possède une gaine dans laquelle une baleine élastique de rappel de posture peut être engagée et deux goussets positionnés à chaque extrémité de la gaine dans lesquels une autre baleine de rappel de posture peut être insérée.

Dans une autre forme de réalisation, l'orthèse comprend une baleine de rappel de posture dont chaque extrémité possède un doigt qui s'insère dans un gousset.

De plus, l'orthèse peut comprendre un plastron de rappel de posture doté de au moins deux doigts et de préférence quatre doigts conçus pour s'insérer dans plusieurs goussets correspondants et de préférence quatre goussets correspondants.

Pour sa bonne compréhension, l'invention est décrite en référence au dessin ci annexé représentant, à titre d'exemple non limitatif, une forme de réalisation d'une orthèse selon celle-ci.
Figure 1 est une vue de la face extérieure d'une orthèse lombaire selon l'invention,
Figure 2 et figure 3 montrent deux formes de réalisation de moyens de réception des éléments de rappel élastique de posture, la seconde étant conforme à la présente invention telle que définie dans la revendication 1,
Figure 4 est une vue en coupe selon IV-IV de figure 3,
Figure 5 montre une autre forme de réalisation d'un orthèse selon l'invention,
Figure 6 montre une autre forme de réalisation d'une orthèse .

Par souci de simplicité, les éléments de l'invention communs aux différentes formes de réalisation portent les mêmes références.

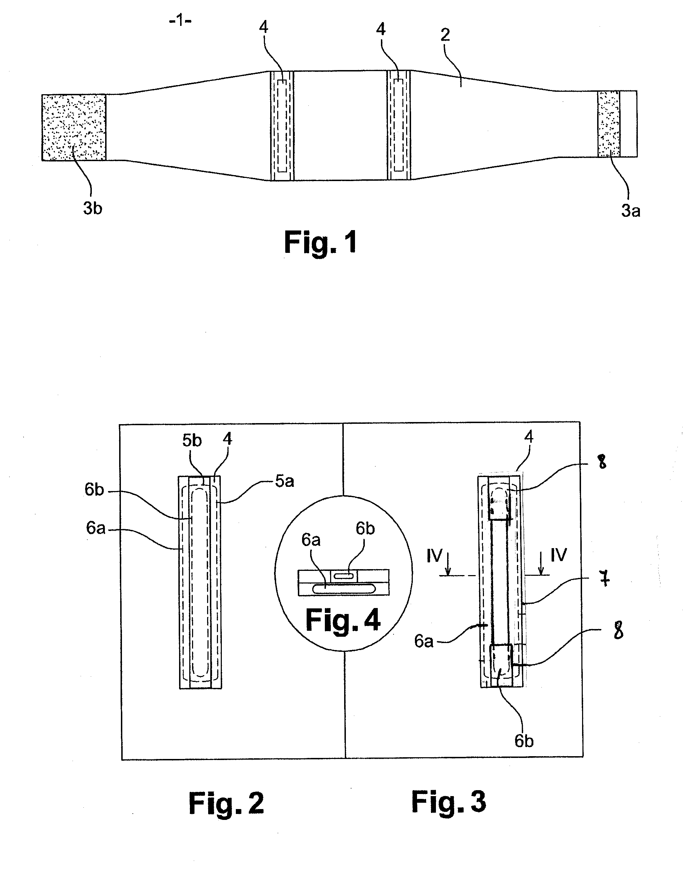

La figure 1 représente une orthèse de soutien lombaire mise à plat en vue extérieure.

Comme on peut le voir sur cette figure, l'orthèse 1 est une ceinture de soutien lombaire qui est constituée d'un corps principal 2 de textile élastique à base, notamment, de fils fortement élastiques.

Il est prévu à chacune des extrémités de la ceinture en question des premiers organes d'attache 3a et des seconds organes d'attache 3b complémentaires permettant de fermer la ceinture sur elle-même autour de la taille d'un utilisateur. Ces moyens d'attache sont, de préférence, des éléments de textile de type crochets à boucles.

De façon conventionnelle, cette ceinture est dotée d'éléments de rappel élastiques de posture.

La ceinture de la figure 1 se distingue des ceintures de l'art antérieur en ce qu'elle est dotée de moyens de rappel de posture évolutifs.

Dans une première forme de réalisation de l'invention telle qu'elle apparaît à la figure 1. Il est prévu de doter la ceinture de deux fourreaux 4 sur la ceinture espacés l'un de l'autre et sensiblement parallèles.

Ces fourreaux 4 ont la particularité de posséder deux gaines 5a et 5b superposées. En pratique, le fourreau 4 comprend trois bandes de textile qui délimitent ainsi deux gaines 5a et 5b.

Avantageusement, ces deux gaines 5a et 5b superposées sont de dimensions inégales. Comme cela apparait sur les figures, le fourreau 4 comprend ainsi une gaine large 5a et une gaine étroite 5b.

Dans l'exemple représenté à la figure 2, les deux gaines 5a et 5b superposées sont intégrées au corps principal 2 par tissage ou par tricotage.

L'avantage apparaît ici clairement dans la mesure où chacune des gaines 5a et 5b peut recevoir une baleine spécifique 6a ou 6b dont les élasticités respectives vont pouvoir s'ajouter ou se retrancher lorsque l'on ôte ou l'on combine l'une ou l'autre des deux baleines 6a ou 6b.

Il est ainsi possible de moduler le rappel de posture en fonction de la ou des baleines qui sont engagées dans la ou les gaines de la bande.

Un rappel de posture maximale est ainsi obtenu lorsqu'une baleine 6a de grande largeur procurant un fort rappel de posture se combine avec une baleine plus étroite 6b offrant un rappel de posture moins important mais qui se combine néanmoins avec la première baleine.

Au fur et à mesure de l'évolution de la pathologie, l'orthèse permet d'effectuer le retrait de la baleine de faible largeur 6b pour ne laisser que la baleine de grande largeur 6a effectuer sa fonction de rappel de posture. Ensuite, il est possible de substituer la baleine à faible largeur 6b à la baleine de grande largeur 6a. Enfin, la baleine de faible largeur 6b est retirée laissant au textile élastique de la ceinture la fonction de rappel de posture et d'immobilisation.

Selon l'invention, il est prévu qu'au moins un fourreau 4 présente une gaine 7 et, à chacune des extrémités, un gousset 8. Par gousset, on entend une pièce de matière textile qui est fermée sur trois de ses côtés et qui définit une cavité.

Ainsi, le rappel de posture est réalisé par une première baleine 6a qui est insérée dans la gaine et par une deuxième baleine 6b. La baleine peut ainsi être engagée au niveau de chacun des goussets 8. La baleine 6b est donc apparente sauf à ses extrémités où elle est inserée dans les goussets 8. Dans cette forme de réalisation, il est possible de mettre en oeuvre la ceinture de contention avec deux baleines combinées ou avec l'une des baleines ou sans aucune baleine au fur et à mesure de l'évolution de la pathologie ou en fonction du port prescrit.

Ainsi, lors d'un port nocturne, il peut être envisagé de porter les deux baleines combinées, tandis que durant un port diurne seulement l'une des baleines sera mise en place dans la ceinture orthopédique.

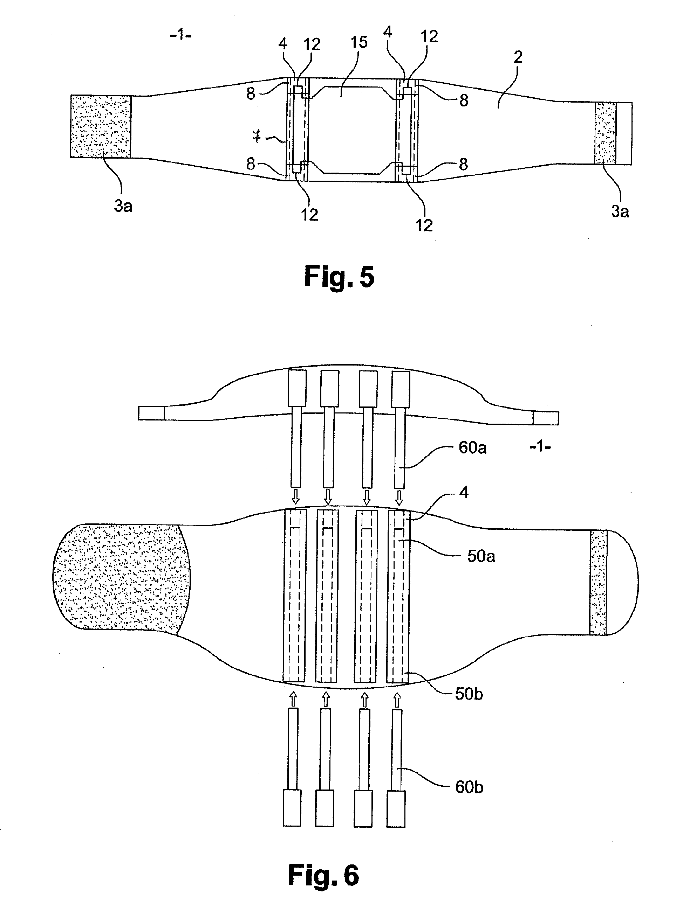

La figure 5 montre une autre forme de réalisation de l'invention dans laquelle la ceinture lombaire est équipée de deux fourreaux 4 telles que ceux qui sont représentées à la figure 3, c'est-à-dire des fourreaux qui présentent, à la fois, une gaine 7 et deux goussets 8 à chaque extrémité du fourreau.

En effet, la ceinture selon l'invention peut recevoir de manière amovible un plastron dorsal 15 qui est lui-même doté de quatre doigts 12 qui viennent s'insérer dans les quatre goussets 8 que l'on trouve sur la ceinture.

Dans une forme de réalisation de l'invention, il est envisagé de doter les baleines 6a qui sont destinées à être retenues dans les goussets 8 de doigts 12 à leurs extrémités pour obtenir une meilleure retenue de ceux-ci dans lesdits goussets 8.

Dans toutes les formes de réalisation que l'on a décrites, il est prévu que chaque bande rapportée soit dotée d'un rabat qui vient se refermer, par exemple, par du tissu à boucles et à crochets de manière à empêcher toute sortie des baleines.

Comme illustré à la figure 6, il est prévu d'adjoindre à l'orthèse un accessoire qui permet d'augmenter de manière sélective la surface de contact avec l'utilisateur.

Comme le montre la figure 6, le corps principal de l'orthèse est doté de quatre fourreaux 4 parallèles qui délimitent deux gaines 50a et 50b. Il est prévu d'équiper l'orthèse d'un plastron 15 dorsal amovible qui peut être réalisé en matière textile avec éventuellement une armature pour lui conférer de la rig id ité.

Le plastron 15 supporte quatre baleines 60a qui permettent de fixer le plastron 17 en insérant les baleines 60a en question dans une gaine 50a du fourreau 4 tandis que des baleines 60b peuvent être insérées indépendamment dans les gaines 50b. Cette forme de réalisation de l'invention s'avère particulièrement avantageuse puisqu'elle permet de combiner une évolutivité non seulement en termes de rappel élastique - par l'effet combiné des baleines - mais également en termes de surface d'appui sur l'utilisateur - par la présence du plastron qui jouxte le corps principal -.

Bien entendu, l'invention n'est pas limitée à la forme de réalisation décrite ci-dessus à titre d'exemple non limitatif, mais elle en embrasse toutes les formes de réalisation. Ainsi, si l'invention a été décrite plus spécialement en référence à une ceinture de soutien lombaire, il peut être tout à fait envisagé qu'elle soit une orthèse de genou, de coude ou de cou.

## Revendications

1. Orthèse (1) orthopédique comprenant un corps principal (2) en matière textile élastique destiné à stabiliser un membre, le corps principal (2) étant associé à des baleines de rappel de posture, ladite orthèse comprenant au moins un fourreau (4) de réception d'au moins deux baleines élastiques (6a,6b) de rappel de posture superposées qui peuvent être mises en place de manière séparée ou combinée, **caractérisée en ce que** ledit au moins un fourreau (4) de réception comprend une bande de textile rapportée sur le corps principal (2) qui possède une gaine (5) dans laquelle la première baleine élastique (6a) de rappel de posture peut être engagée et deux goussets (8) positionnés à chaque extrémité de la gaine dans lesquels la seconde baleine élastique (6b) de rappel de posture peut être insérée.

2. Orthèse orthopédique selon la revendication 1, **caractérisée en ce que** ladite orthèse comprend au moins deux fourreaux (4) de textile disposés à distance l'un de l'autre.

3. Orthèse orthopédique selon la revendication 1 ou la revendication 2, **caractérisée en ce qu'**au moins un fourreau (4) de réception est constitué d'au moins deux bandes de textile superposées fixées sur le corps principal de manière à délimiter au moins deux gaines (5a, 5b) dans chacune desquelles une baleine élastique (6a, 6b) de rappel de posture peut être indépendamment engagée.

4. Orthèse orthopédique selon l'une des revendications 1 à 2, **caractérisée en ce qu'**au moins un fourreau (4) intègre au moins deux gaines (5a,5b) qui présentent des dimensions différentes de manière à recevoir des baleines (6a,6b) de rappel de posture de caractéristiques dimensionnelles et constructives différentes.

5. Orthèse orthopédique selon l'une des revendications 1 à 4, **caractérisée en ce que** l'orthèse comprend un plastron (17) équipé d'au moins deux baleines de rappel (60a) conçues pour s'engager sur une gaine (50a) de deux fourreaux de manière à juxtaposer le plastron (17) et le corps principal (2) pour augmenter la surface d'appui de l'orthèse.

6. Orthèse orthopédique selon l'une des revendications 1 à 5, caractérisée en ce l'orthèse comprend une baleine de rappel de posture dont chaque extrémité possède un doigt (12) qui s'insère dans un gousset (8).

7. Orthèse orthopédique selon la revendication 6 lorsqu'elle est dépendante de la revendication 4, **caractérisée en ce que** l'orthèse comprend un plastron (15) de rappel de posture doté de au moins deux doigts (12) et de préférence quatre doigts (12) conçus pour s'insérer dans plusieurs goussets (8) correspondants et de préférence quatre goussets (8) correspondants.

## Patentansprüche

1. Orthopädische Orthese (1), die einen Hauptkörper (2) aus einem elastischen Textilmaterial umfasst, der dazu vorgesehen ist, ein Körperglied zu stabilisieren, wobei der Hauptkörper (2) mit Haltungsrückstellstäbchen verbunden ist, wobei die besagte Orthese mindestens ein Futteral (4) zur Aufnahme von mindestens zwei übereinander gelegten elastischen Haltungsrückstellstäbchen (6a, 6b) umfasst, die getrennt oder kombiniert eingesetzt werden können, **dadurch gekennzeichnet, dass** das besagte mindestens eine Aufnahmefutteral (4) ein Textilband umfasst, das auf dem Hauptkörper (2) angefügt ist und das über eine Scheide (5), in die das erste elastische Haltungsrückstellstäbchen (6a) eingebracht sein kann, und zwei Taschen (8) verfügt, die an jedem Ende der Scheide positioniert sind und in die das zweite elastische Haltungsrückstellstäbchen (6b) eingefügt werden kann.

2. Orthopädische Orthese nach Anspruch 1, **dadurch gekennzeichnet, dass** die besagte Orthese mindestens zwei Textilfutterale (4) umfasst, die voneinander beabstandet angeordnet sind.

3. Orthopädische Orthese nach Anspruch 1 oder 2, **dadurch gekennzeichnet, dass** mindestens ein Aufnahmefutteral (4) aus mindestens zwei übereinander gelegten Textilbändern besteht, die derart auf dem Hauptkörper befestigt sind, dass sie mindestens zwei Scheiden (5a, 5b) begrenzen, in die jeweils ein elastisches Haltungsrückstellstäbchen (6a, 6b) unabhängig eingebracht sein kann.

4. Orthopädische Orthese nach einem der Ansprüche 1 bis 2, **dadurch gekennzeichnet, dass** mindestens ein Futteral (4) mindestens zwei Scheiden (5a, 5b) integriert, die unterschiedliche Abmessungen aufweisen, um Haltungsrückstellstäbchen (6a, 6b) mit unterschiedlichen Abmessungs- und Konstruktionscharakteristika aufzunehmen.

5. Orthopädische Orthese nach einem der Ansprüche 1 bis 4, **dadurch gekennzeichnet, dass** die Orthese eine Frontplatte (17) umfasst, die mit mindestens zwei Rückstellstäbchen (60a) ausgestattet ist, die dazu entworfen sind, mit einer Scheide (50a) von zwei Futteralen derart in Eingriff zu kommen, dass die Frontplatte (17) und der Hauptkörper (2) aneinander lagern, um die Auflagefläche der Orthese zu vergrößern.

6. Orthopädische Orthese nach einem der Ansprüche 1 bis 5, **dadurch gekennzeichnet, dass** die Orthese ein Haltungsrückstellstäbchen umfasst, von dem jedes Ende über einen Finger (12) verfügt, der sich in eine Tasche (8) einfügt.

7. Orthopädische Orthese nach Anspruch 6, wenn dieser von Anspruch 4 abhängig ist, **dadurch gekennzeichnet, dass** die Orthese eine Haltungsrückstellfrontplatte (15) umfasst, die mit mindestens zwei Fingern (12) und vorzugsweise vier Fingern (12) versehen ist, die dazu entworfen sind, sich in mehrere entsprechende Taschen (8) und vorzugsweise vier entsprechende Taschen (8) einzufügen.

## Claims

1. An orthopedic orthosis comprising a main body (2) in an elastic textile material intended for stabilizing a member, the main body (2) being associated with pre-shaped posture stays, said orthosis comprising at least a sleeve (4) for receiving at least two superimposed elastic pre-shaped posture stays (6a, 6b) which may be placed separately or in a combined manner, **characterized in that** said at least one receiving sleeve (4) comprises a textile strip added onto the main body (2) which has a sheath (5) in which the first elastic pre-shaped posture stay (6a) may be engaged and two gussets (8) positioned at each end of the sheath in which the second elastic pre-shaped posture stay (6b) may be inserted.

2. The orthopedic orthosis according to claim 1, **characterized in that** said orthosis comprises at least two textile sleeves (4) disposed at a distance from each other.

3. The orthopedic orthosis according to claim 1 or claim 2, **characterized in that** at least one receiving sleeve (4) is constituted of at least two superimposed textile strips secured onto the main body in such a manner as to delimit at least two sheaths (5a, 5b) in each of which an elastic pre-shaped posture stay (6a, 6b) may be independently engaged.

4. The orthopedic orthosis according to any of claims 1 to 2, **characterized in that** at least one sleeve (4) integrates at least two sheaths (5a, 5b) which have different dimensions in such a manner as to receive pre-shaped posture stays (6a,6b) of different dimensional and constructive features.

5. The orthopedic orthosis according to any of claims 1 to 4, **characterized in that** the orthosis comprises a panel (17) equipped with at least two pre-shaped stays (60a) designed to engage on a sheath (50a) of two sleeves in such a manner as to juxtapose the panel (17) with the main body (2) in order to increase the bearing surface of the orthosis.

6. The orthopedic orthosis according to any of claims 1 to 5, **characterized in that** the orthosis comprises a pre-shaped posture stay of which each end has a finger (12) which is inserted in a gusset (8).

7. The orthopedic orthosis according to claim 6 when it is dependent on claim 4, **characterized in that** the orthosis comprises a pre-shaped posture panel (15) fitted with at least two fingers (12) and preferably four fingers (12) designed to be inserted into several corresponding gussets (8) and preferably four corresponding gussets (8).
